# EUROPEAN PATENT SPECIFICATION

(11) **EP 2 591 802 B1**
(45) Date of publication and mention of the grant of the patent: **08.06.2016**
(21) Application number: 11743950.5
(22) Date of filing: 05.07.2011
(51) Int. Cl.: A61K 39/39, A61P 31/06, A61P 31/18, A61P 31/00, A61P 35/00, A61K 9/127

(54) **VACCINE COMPOSITIONS BASED ON STICHOLYSIN ENCAPSULATED IN LIPOSOMES**
IMPFSTOFFZUSAMMENSETZUNG, ENTHALTEND STICHOLYSIN, EINGEKAPSELT IN LIPOSOME
COMPOSITION VACCINALE,CONTENANT STICHOLYSIN ENCAPSULÉ DANS DES LIPOSOMES

(30) Priority: 06.07.2010 WO PCT/CU2010/000144
(43) Date of publication of application: 15.05.2013
(73) Proprietor: Centro de Inmunologia Molecular, La Habana 12100 (CU)
(72) Inventor: FERNANDEZ MOLINA, Luis Enrique, La Habana 12100 (CU); LANIO RUIZ, Maria Eliana, La Habana 11100 (CU); LABORDE QUINTANA, Rady Judith, La Habana 1200 (CU); CRUZ LEAL, Yoelys, La Habana 10600 (CU); LUZARDO LORENZO, Maria del Carmen, La Habana 13200 (CU); MESA PARDILLO, Circe, La Habana 10400 (CU); ALVAREZ VALCARCEL, Carlos Manuel, La Habana 10400 (CU); PAZOS SANTOS, Isabel Fabiola, La Habana 10400 (CU); TEJUCA MARTINEZ, Mayra, La Habana 11300 (CU); VALLE GARAY, Aisel, La Habana 17100 (CU); ALONSO BIOSCA, Maria Eugenia, La Habana 10400 (CU); CANET SANTOS, Liem, La Habana 11300 (CU)
(74) Representative: V.O.
(86) International application number: PCT/CU2011/000004
(87) International publication number: WO 2012/003814

(56) References cited:
- DIETRICH G ET AL: "From evil to good: a cytolysin in vaccine development", TRENDS IN MICROBIOLOGY, ELSEVIER SCIENCE LTD., KIDLINGTON, GB, vol. 9, no. 1, 1 January 2001 (2001-01-01) , pages 23-28, XP002382792, ISSN: 0966-842X, DOI: 10.1016/S0966-842X(00)01893-X cited in the application
- ALVAREZ CARLOS ET AL: "Sticholysins, two pore-forming toxins produced by the Caribbean Sea anemone Stichodactyla helianthus: their interaction with membranes.", TOXICON : OFFICIAL JOURNAL OF THE INTERNATIONAL SOCIETY ON TOXINOLOGY 15 DEC 2009 LNKD- PUBMED:19268489, vol. 54, no. 8, 15 December 2009 (2009-12-15), pages 1135-1147, XP002660719, ISSN: 1879-3150 cited in the application
- MARTINEZ ET AL: "Effect of sphingomyelin and cholesterol on the interaction of St II with lipidic interfaces", TOXICON, ELMSFORD, NY, US, vol. 49, no. 1, 15 December 2006 (2006-12-15), pages 68-81, XP5803971, ISSN: 0041-0101, DOI: 10.1016/J.TOXICON.2006.09.019 cited in the application
- LANIO M E ET AL: "Humoral immune response against epidermal growth factor encapsulated in dehydration rehydration vesicles of different phospholipid composition.", JOURNAL OF LIPOSOME RESEARCH 2008 LNKD- PUBMED:18348068, vol. 18, no. 1, 2008, pages 1-19, XP22350779, ISSN: 1532-2394 cited in the application

## Description

### TECHNICAL FIELD:

The present invention relates to the field of biotechnology applied to human health. Particularly, the present invention relates to a vaccine vehicle for use in both subcutaneous and intramuscular based on liposomes containing sticholysin and enhancing antigen-specific immune cellular responses, useful in cancer immunotherapy and treatment of diseases caused by intracellular pathogens.

### PRIOR ART:

The immunoadjuvant capacity of the liposomal vesicles has long been known.

Rationality that exists in the use of liposomes in immunization and vaccine design is based on their ability to release antigenic molecule on antigen presenting cells (APC) and stimulate an immune response. The most important advantages of liposomes as immunoadjuvants are summarized in: (i) the ability to mimic pathogens carrying large amounts of antigen to APC, (ii) the possibility of co-encapsulating antigens with immunostimulatory molecules, (iii) the flexibility to modify its physicochemical properties for the purpose of more effective, and (iv) the fact of being biodegradable and nontoxic (Leserman in Journal of Liposome Research, 2004, 14 (3 & 4); 175-189).

A challenge in the field of vaccinology is the enhancement of cellular immune response mediated by antigen-specific cytotoxic T lymphocytes CD8+ (CTL), with relevance for the prevention and treatment of diseases induced by intracellular pathogens and tumor cells. Liposomal vesicles can enhance a CTL response but not always effectively.

Different strategies based on liposomal vesicles have been designed with the intention of streamlining this function; examples are acidic pH-sensitive liposomes, cationic liposomes, the inclusion of immunomodulators such as CpG and pore-forming toxins form bacteria. Despite the variety of publications, some of these strategies have had limited success in the induction of effective cellular immune responses or are disadvantaged and therefore require a better design before implementation.

The integration of viral membrane proteins in the liposomal membrane in order to promote membrane fusion under conditions of acid pH or proteolytic processing, has been another alternative for the development of vaccine vehicles. These vesicles known as virosomes have not only been used as parental virus vaccines, but have also been exploited as vehicles for vaccine antigens, bound or encapsulated to virosome (Zurbriggen en Vaccine, 2003 14; 21(9-10):921-4). Virosome particles known with abbreviation IRIV (*immunopotentiating reconstituted influenza virosomes*), containing proteins and lipids from the envelope of influenza virus, are the best example of this strategy and form the basis of the patent of the vaccine against hepatitis A (United States Patent 5565203). However, this vaccine preparation was designed to enhance neutralizing antibody response against hepatitis A. Binding of hepatitis A virus inactivated and highly pure in the virosome membrane favored the processing and presentation of derived peptides by the classical pathway MHCII, as a result of the fusion process of the virosome and endosomal membranes in APCs (Glück and Wälti in Dev Biol (Basel), 2000, 103,189-97). The evidence described suggest that antigen-virosomes physical association is important in the immunoadjuvant (Zurbriggen et al., Progr., in Lipid Res., 2000, 39(1), 3-18; Amacker et al., in Int Immunol., 2005, 17(6), 695-704).

Schumacher et al., in Vaccine 22:714-723, 2004, reported that IRIVs are able to enhance cellular mediated immune response. Particularly, Schumacher *et al.* demonstrated its adjuvant activity in the induction of CTL *in vitro.* This ability depended mainly on the stimulation of the reactivity of CD4 + T cells specific to viral proteins.

However, although the use of virosomes as adjuvants has many advantages such as low toxicity and high immunogenicity, one of the problems in current virosomal technology is the absence of procedures for the efficient encapsulation of solutes such as proteins, required for induction of a CTL response. A lipid concentration at which virosomes are produced (1 mM of lipid, approximately), and considering its diameter (about 200 nm), less than 1% of the aqueous phase is encapsulated within the virosomes (Schoen et al., en J. Liposome Res., 3: 767-792, 1993). These features significantly reduce the efficiency of virosomes to release antigens or genes to cells.

One strategy to overcome this limitation and to produce immunogenic preparation for CD8 + T cells, recently published in the U.S. patent application number 20100015214, is based on a combination of empty virosomes with vehicles, preferably liposomes, which encapsulate antigens. U.S. 20100015214 discloses a trans-adjuvant effect of virosomes, although these particles and the liposomes do not exhibit any physical interaction between them.

In the article by Lanio. et al. published in the J Liposome Res.,2008,18(1),1-19 is described, for rhEGF, higher encapsulation-retention efficiency of liposome obtained by the dehydration-rehydration procedure (DRVs) and comprised of phosphatidylcholine (PC) saturated (dipalmitoylphosphatidylcholine, DPPC) and cholesterol (Cho) in molar ratio 1:1 compared with those containing unsaturated PC and Cho. In fact, the procedure for obtaining DRVs yields multi-bilayer vesicles with high encapsulation/ retention efficiency for a wide variety of soluble solutes. Lanio *et al.* demonstrated the immunoadjuvant propierties of these vesicles to enhance an antibody response quantitatively and qualitatively superior against rhEGF.

The review published by Alvarez et al, in Toxicon, 2009, 54(8):1135-47, summarizes the structural and functional characteristics of two proteins produced by a marine invertebrate, the Caribbean Sea anemone *Stichodactyla helianthus,* and named by the authors Sticholysins (Sts) I and II (StI/II). These proteins are pore-forming toxins (PFTs) belonging to the protein family *Actinoporins,* unique classes of PFTs of eukaryotic origin found exclusively on sea anemones. Similar to other members of this family, Sts are basic proteins with high isolelectric point (>9.5), with molecular mass of approximately 20 kDa, devoid of cysteine residues in their amino acid sequences and exhibiting a preference for membranes containing sphingomyelin (SM). Sts are produced in soluble form but can easily associate with different cellular and model membrane systems forming pores with a diameter of 2 nm, probably due to the interaction of the N-terminal's α helices from four monomers. Both events, the association and pore formation, depend on the physicochemical properties of membranes.

In the article published in Toxicon, 2007, 49: 68-81, Martinez *et al.* reports the relevance of the presence of SM and Cho for the association and pore-formation in the membranes by Stll. The phase state of the membrane influences the StII association with these structures. As reported by Martinez et al., in the journal Biology, 2002, 16, 2: 85-93, this toxin is associated reversibly to membranes of DPPC and SM, with a very low capacity of permeabilization.

Although widely reported the immunomodulatory properties of pore-forming toxins from bacteria for the induction of antigen-specific CTL response, using different strategies including their encapsulation into liposomes, as summarized in reviews by Dietrich *et al.* and Morón et al., in TRENDS in Microbiology, 2001, Vol.9 No.1, 23-28 and TRENDS in Immunology, 2004, Vol.25 No.2, 92-97, respectively; nothing has been referred to the functionally homologous toxins from marine eukaryotic organisms. From the point of view of their applications in immune therapy of tumor diseases, it is desirable to have new vaccine formulations able of inducing specific cellular immune response to antigens and are less aggressive than those compositions containing bacterial toxins.

### BRIEF DESCRIPTION OF THE INVENTION:

Surprisingly, the authors of the present invention have found that when an antigen is administered subcutaneously (sc) or intramuscularly (im) encapsulated in the vaccine formulation Liposome-St, with any of the variants of toxins described including those that do not exhibit pore-forming or lytic activity, much higher percentages of lysis of target cells are induced than those observed in the positive control group using Polyinosinic:polycytidylic acid (poly I:C, PIC) which is considered in the state of the art as the quintessential enhancer of CTL response, unmodified antibody levels and mixed Th1/Th2 response induced by the liposomal antigen. This indicates the potential of liposome-St system to generate both cellular and humoral response against a protein antigen, which is relevant to the use of this formulation for vaccination purposes.

The object of this invention is a vaccine vehicle as described in claim 1. Liposomal preparations of DPPC and Cho in which are encapsulated mutants of toxins known as Sts (eg StIW111C and StIW111Cirrev) together with protein antigen are also the subject of the present invention.

In another aspect, the vaccine compositions based on this vaccinal vehicle containing the toxins together with an antigen administered sc or im for induction of a strong antigen-specific CTL response are also the subject of the present invention.

Further described herein is the use of the vaccinal compositions described, based on this vaccine vehicle containing the toxins together with an antigen and administered sc or im for strengthening of the immune response of a patient suffering from a disease selected from the group consisting of cancer and infectious diseases caused by intracellular pathogens.

Herein described is also a method for treating patients who require a strengthening of their immune response, wherein the patient suffers from a disease selected from the group consisting of cancer and infectious diseases caused by intracellular pathogens and wherein the method comprises administering sc or im the vaccinal compositions based on this vaccine vehicle containing the toxins Sts or their mutants together with a relevant antigen for the disease.

Herein is also described the protection provided by such vaccine compositions to the challenge with tumor cells expressing the antigen, reducing the percentage of tumors and increasing survival of subjects with tumors. Therefore, a prophylactic method of onset of illness related to the antigen is also described herein.

### DETAILED DESCRIPTION OF THE INVENTION

The vaccine vehicle of the invention is based on aqueous dispersions of DRV liposomes of DPPC:Cho: in a 1:1 molar ratio that encapsulate St together with antigen, and eventually can contain other co-solvents miscible with water, no toxic, non-irritating and do not cause destabilization of the vesicles, such as those commonly used in injectable pharmaceutical compositions, for example sugars, polyethylene glycol, etc.

Liposomal preparations of the present invention are obtained by the dehydration-rehydration procedure and constituted by DPPC and Cho in a 1:1 molar ratio and co-encapsulating an antigen with some of the Sts variants or their reversibly inactive mutants, wherein said vaccinal compositions are able to promote an antigen-specific CTL immune response and protective against challenge with tumor cells.

In one embodiment of the invention the vaccine composition contains as antigen the protein OVA, but wherein the antigen can be any protein or polypeptide associated to a disease for which, from a therapeutic standpoint, is relevant induce a specific CTL immune response against this protein antigen. For example, the antigen may be a protein or polypeptide associated with cancer, a protein or polypeptide associated with AIDS or a protein or polypeptide associated with tuberculosis.

The liposomes decribed herein are obtained by dehydration-rehydration technology reported by Kirby and Gregoriadis in Biotechnology, 1984, 2, 979-984, these are vesicles with an internal aqueous phase surrounded by several lipid bilayers characterized by high efficiency of encapsulation and retention of labile molecules such as antigen and immunomodulator proteins. Chloroform solutions of DPPC and Cho in a 1:1 molar ratio are evaporated and kept under vacuum for 30 minutes. The lipids were hydrated with deionized water at a temperature above the phase transition temperature (Tc) of DPPC (T> 45 °C). Suspensions of multilamellar vesicles resulting (MLVs) are transformed in SUV or LUV (*Small Unilamellar Vesicles* and *Large Unilamellar Vesicles,* respectively) by ultrasound and rest cycles or extrusion through polycarbonate membranes of pore size 100 nm. The vesicles obtained were mixed with protein solutions, following a relationship lipid: protein from 16 - 64 µmol of lipids: 1.8 -7 nmol of antigen and 0.5 - 2 nmol of St, and lyophilized for 20-24 hours. Rehydration was performed with deionized water and stirring for 30 minutes at a temperature less than 45 ° C. The material unencapsulated in liposomes is removed by washing with phosphate buffered saline (PBS) pH 7.4 (NaCl 136 mmol/L, KH₂PO₄ 1.47 mmol/L, Na2HPO4 9.55 mmol/L, KCI 2.68 mmol/L) followed by centrifugation at 10 000 g for 15 min. Such preparations reach encapsulation efficiency for the different variants of Sts ranging around 50% with retention of 70% after one month of storage at 4 ° C suspended in PBS.

Stl and Stll toxins are isolated and purified from the sea anemone *Stichodactyla helianthus,* using the procedure described by Lanio et al. in Toxicon. 2001, 39, 187-94. The recombinant Sticholysin I (rStl) and rStl mutant, Stl W111C, are obtained according to the procedures described by Pazos et al. in Toxicon, 2006, 48, 1083-1094 and Pent6n et al. in Protein Eng Des Sel. 2011, 24, 485-493, respectively.

The concentration of Sts used in the vaccine vehicle of the present invention is in the range of 0.25 to 1 µM co-encapulated with antigen in a concentration range of 1 - 3.5 µM in a liposomal suspension with a range of total lipid concentration of 16 to 64 mM.

Preferably the vaccine vehicle of the present invention contains an amount of Sts of 0.3 to 0.4 nmol co-encapulated with 1 to 2 nmol of antigen in a liposomal suspension of 20 µmo) of total lipids in a volume of 200 µL.

The dose range used for the vaccines referred to in the present invention, by sc or im, is 1 - 2 nmol (50-100 mg) of antigen.

A key feature of the vaccine compositions of the invention is that they lack immunological adjuvants other than those described.

As already explained above, it is known from the prior art that bacterial pore-forming toxins encapsulated into liposomal vesicles are able to enhance a CTL response, but it is known that use of vaccine compositions based on bacterial toxins have adverse effects on human; however, the authors of the present invention have found unexpectedly that non-bacterial toxins are able to have the same immunostimulator effect. Moreover, the authors of this invention have managed to decouple the pore-forming activity of the toxins from their stimulator effects of the CTL response, which offers great advantages for clinical use in humans to this vaccine vehicle. The results obtained using in the vaccine vehicle of the present invention the molecular entities StIW111Cᵢᵣᵣₑᵥ or heat-inactivated Stll co-encapsulated with antigen into liposomes, demonstrate that there is no absolute dependence between the enhancement of an antigen-specific CTL immune response by the formulations of liposome-St and the ability of these proteins to form pores in membranes. The test of maturation of dendritic cells (DCs) isolated from bone marrow of C57BL/6 mice and exposed to Stll *in vitro,* shows the ability of this protein not only in its active variant, but also in that heat-inactivated, to induce the activation of CDs, similar to that seen with LPS (positive control) under similar conditions.

The ability of Sts to form pores in membranes was assessed by testing hemolytic activity and permeability of LUVs loaded with carboxyfluorescein (CF) as described by Martínez et al. in Toxicon, 2001, 39,1547-1560. It was shown that Sts do not exhibit permeabilizing activity by analyzing permeability of liposomal vesicles of DPPC:Cho (1:1) encapsulating CF, compared with that observed in vesicles composed of egg yolk phosphatidylcholine and SM (1:1) (positive control).

It was verified that the mutant StIW111C forms an inactive dimer stabilized by a disulfide bond by the hemolytic activity assay and SDS-PAGE in the presence and absence of 2-mercaptoethanol (2-ME) as reducing agent, just as described by Pent6n et al. in Protein Eng Des Sel. 2011, 24, 485-493. The inability of dimeric structure to form pores in erythrocytes, as model cells, was result of its non-association with membrane.

A procedure to obtain an irreversible dimeric specie of StIW111C (StIW111Cᵢᵣᵣₑᵥ) based on the reduction of StIW111C by incubation with 2-ME (0.1 mol/L), elimination of reducing agent by filtration on column PD-10, and immediate incubation of StIW111Cₘₒₙₒₘₑᵣ with the homobifunctional reagent bis (maleimide) hexane (BMH) in a molar ratio StIW111Cₘₒₙₒₘₑᵣ:BMH 1:1 6 2:1 for 2 hours at 4 ° C, was established. StIW111Cᵢᵣᵣₑᵥ was purified by gel filtration chromatography on column Superdex 75 HR 10/300 in the presence of a reducing agent. The obtaining of the irreversible dimer and its purity degree were verified by SDS-PAGE under reducing conditions. The irreversible dimer was obtained with 94% purity and only 6% contaminant of the reversible dimer. The absence of functional activity was verified by hemolytic activity assay.

Stll irreversible inactivation by heat treatment was performed according to the procedure described by Martínez et al. in Toxicon, 2001, 39,1547-1560 and the total loss of the ability to form pores in the membrane was checked by hemolytic activity assay.

The immunomodulator properties of the different molecular species Sts in the system liposome-St related to its ability to enhance an antigen-specific cytotoxic immune response *in vivo* and antitumor immunity in a preventive scenary, can be measured in the experimental model of the mice strain C57BL/6 using the antigen OVA and the tumor cell line E.G7, a subclone of murine EL-4 thymoma (Kusmartsev and Gabrilovich, in J Leukoc Biol., 2003, 74: 186-96), transfected with complementary DNA of OVA pAc-neo-OVA (Moore, et al. in Cell, 1988, 54: 777-85) and obtained from *"American Type Cultures Collection"* (ATCC, VA).

The authors of the present invention have also found that preventive inoculation with the vaccinal vehicle liposomes-St containing antigen induces antitumor protection higher than that generated by liposomes containing no StII.

These results demonstrate that the system liposome-St is effective to induce an antitumor response functionally robust and protective, when using a protein antigen, without the need for other adjuvants. In turn, the use of this preparation in combining therapies may further enhance its antitumor effect.

In the following examples it is included comparative experimental details that allow verifiyng the immunological efficacy of inducing an antigen-specific cytotoxic immune response *in vivo* and antitumor immunity in a preventive scenary of the vaccinal compositions object of the invention with regard to the other non-liposomal formulations and do not contain Sts.

### EXAMPLES:

### Example 1: Assessment of cytotoxicity and CTL response of the vaccinal vehicle using native Stl or StII and OVA as protein antigen.

The vaccinal vehicle based on liposomes was prepared as previously described.

In DRVs vesicles comprised of DPPC and Cho (molar ratio1:1), OVA as model antigen and Sts (Stl or Stll) were co-encapsulated in a 10 µmol of total lipids: 1.1 nmol OVA and 0.3 nmol of St molar ratio in phosphate buffer saline (PBS) pH 7.4. Following this procedure, two vacinal compositions were obtained:
Vaccinal Composition A: DRVs liposomes of DPPC:Cho (60 µmol of total lipids) encapsulating 6.6 nmol (50 µg) of OVA.
Vaccinal Composition B: DRVs liposomes of DPPC:Cho (60 µmol of total lipids) co-encapsulating 6.6 nmol (50 µg) of OVA and 1.88 nmol of StI or StII.

Fifteen C57BL/6 female mice with a body weight ranging from 18-20 g were selected and separated into 5 experimental groups of three animales each.

Group 1 (negative control) was inoculate by sc route on days 0, 12, 13 and
14, with 0.2 mL phosphate buffered saline (PBS).

Group 2 (positive control) was inoculated by sc route, on day 12, with 22.2 nmol (1 mg) of OVA and mixtured with 100 µg of polyinosinoic- polycitidilic acid (PIC), a TLR3 synthetic ligand and a classical inductor of CTL response (Hamilton-Williams et al. in J. Immunol., 2005, 174: 1159-63), on days 13 and 14 the animals received again 100 µg of PIC.

Group 3 was inoculated by sc route on days 0 and 12, with 0.2 mL of vaccinal composition A (equivalent to a 1.1 nmol or 50 µg of OVA).

Group 4 was inoculated by subcutaneous route on days 0 and 12, with 0.2 mL of vaccinal composition B (equivalent to 1.1 nmol or 50 µg of OVA and 0.3 nmol or 6.25 µg of Stl).

Group 5 was inoculated by sc route on days 0 and 12, with 0.2 mL of vaccinal composition B (equivalent to 1.1 nmol or 50 µg of OVA and 0.3 nmol or 6,25 µg of StII). On day 20 of the experiment, spleen cells from C57BU6 non-immunized mice were incubated with two concentrations of carboxy-fluorescein diacetate succinimidyl ester (CFSE) (0.33 and 5 µmol/L, respectively); the labeled cells with the highest fluorescence intensity were also incubated with 1 µmol/L of OVA (257-SIINFEKL-264) immunodominant peptide in the context of the MHC I haplotype for C57BL/6 mice strain. Afterwards, both populations of labeled cells were mixed 1:1 and the experimental groups 1-5 were inoculated by the tail vein with 30 x10⁶ cells of the mixture in 0.2 mL total volume. Mice were sacrificed after 16 hours and lysis (%) of the target cells was determined in the inguinal lymph node closer to the immunization site by flow cytometry (FACS).

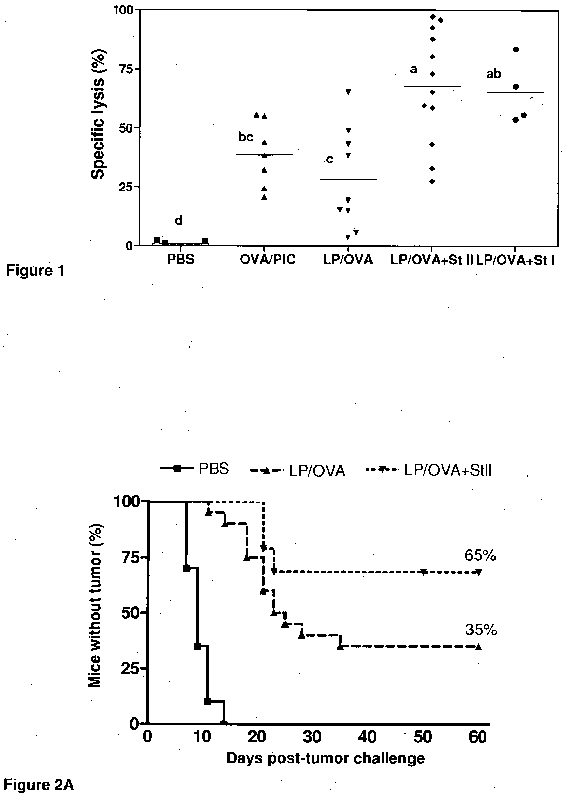

Figure 1 shows the cytotoxicity produced *in vivo* by immunization of mice with liposomes co-encapsulating OVA and Sts. The immunized animals with liposomes co-encapsulating Sts (StI or Stll) showed a CD8+ T cytotoxic lymphocyte response (CTL) specific to OVA stastically higher than the positive control group. Additionally, liposomes that only contained OVA also induced a CTL response statistically similar to the classical positive control for this assay (PIC).

### Example 2: Induction of antitumoral protection of the vaccinal vehicle in the OVA protein model.

The ability of the liposome-based vaccines to induce antitumoral protection was studied. To this end, sixty C57BL/6 female mice with a body weight ranging between 18-20 g were selected and separated into 3 assay groups of 20 animals each.

Group 1 (negative control) was inoculated by im route, on days 0 and 12, with 0.2 mL of phosphate buffer saline (PBS).

Group 2 was inoculated by im route, on days 0 and 12, with 0.2 mL of the vaccinal composition A described in example 1 (equivalent to 1.1 nmol or 50 µg of OVA).

Group 3 was inoculated by im route, on days 0 and 12, with 0.2 mL of the vacinal composition B described in example 1 (equivalent to 1.1 nmol or 50 µg of OVA and 0.3 nmol or 6.25 µg of StII).

All the Groups 1 to 3 were challenges on day 19 with 3x10⁵ cells from the tumor line E.G7 by subcutaneous route (0.2 mL).

Animals were individualized from day 0 and the following parameters were determined thrice per week: tumor volume, time to progression and survival.

The results obtained are described below:

### Time to progression

The time to progression is a parameter that characterizes the time elapsed for each animal from the moment the tumor is inoculated until its appearance. As for mice that had not developed a tumor at the end of the experiment, it was considered that the time to progression was 60 days. The impact on the extension of the time to progression is a highly desirable parameter for a vaccine against cancer. As it can be observed in Figure 2A, animals from group 3, vaccinated with the formulation vaccinal composition B described in Example 1 and object of the invention, showed the most outstanding results

### Survival

This parameter assesses the ability of vaccination to increase the time that immunized animals live upon challenged with the OVA-expressing tumor cells (E.G7). This parameter is measured in days and has a relative character, since it is compared with survival of non-treated animals. In order to prove the statistical significance of the differences found in survival results among groups the Log-Rank test was used.

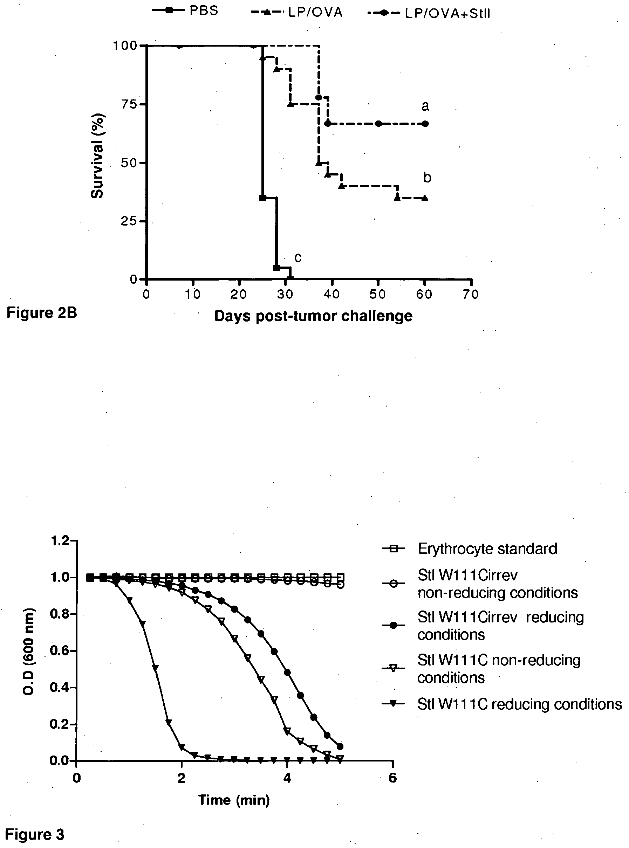

Figure 2B clearly shows that animals from group 3, vaccinated with the vaccinal composition B described in example 1 and object of the present invention are those that survive longer after tumor inoculation.

### Example 3: Hemolytic Activity of Sts mutants.

Pore-formation in erythrocyte membrane produces a colloid osmotic shock that brings about cell lysis. Pore-forming ability of the so called porins can be followed by its hemoleytic activity (HA) which can be experimentally determined by measuring the loss of apparent absorbance (λ = 600 nm) of an erythrocyte suspension due to cell lysis.

In the assay, the HA of the StIW111C irreversibly inactive dimer (StI W111Cirrev) was compared with that of the reversible dimer StIW111C, in reducing (2-ME 0.1 mol/l) and non-reducing conditions, at a relatively high protein concentration in the assay (0.15 µmol/l), if compare with the HA of Stl/Stll reported by Álvarez et al. in Toxicon, 2009, 54(8):1135-47. The time-courses of hemolysis shown in Figure 3 indicate that StIW111Cᵢᵣᵣₑᵥ was inactive under non-reducing conditions. Under reducing conditions, StIW111Cᵢᵣᵣₑᵥ showed less activity than StIW111C either completely reduced or nonreduced.

### Example 4: Assessment of the pore-forming ability of inactivated StII.

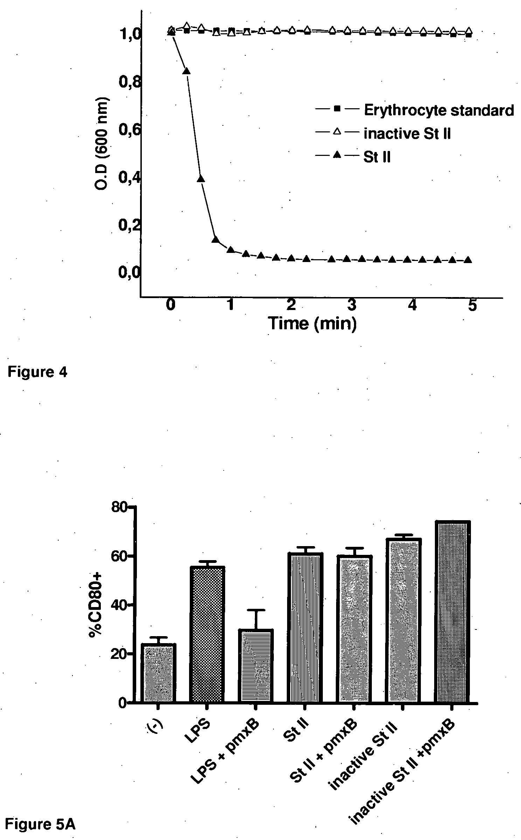

The loss of the ability to form pores in membranes by StII irreversibly inactivated by heating at 80 ° C for two hours was registered using the hemolytic activity test. Figure 4 shows the lack of hemolytic activity for the thermal-inactivated Stll when compared to the active protein.

### Example 5. Effect of the vaccinal vehicle on DCs maturation.

Maturation of DCs induced by StII was assessed in cells obtained from the bone marrow of C57BL/6 mice exposed to active StII (0.1 nmol or 2 µg) or inactivated by thermal treatment (4 µg) in the presence or not of 20 µg of polymyxin B (pmxB, a neutralizing agent of endotoxin's biological activity by binding to lipid A fraction of LPS), for 24 hours at 37 ° C in a 5% CO₂ chamber.

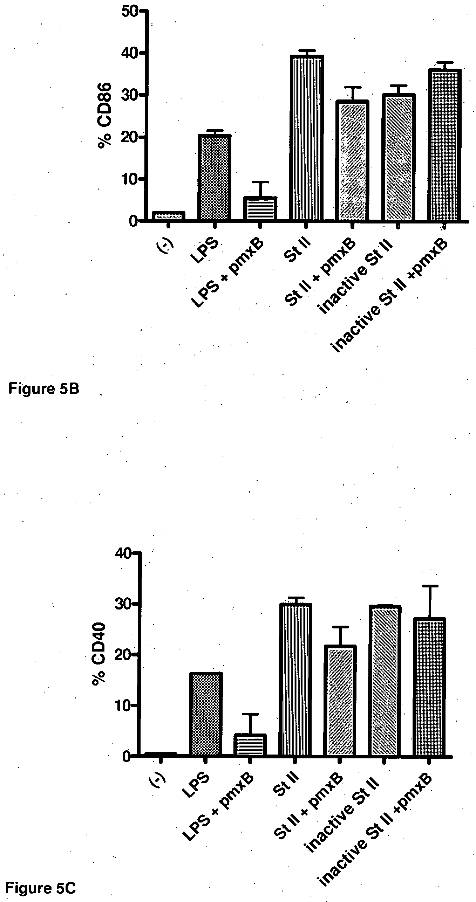

As positive control, it was used LPS (2 µg) and the negative control was RPMI medium plus 30 pmxB. Cells extracted from mice bone marrow were cultured in a number of 600 000 DCs precursors on RPMI per well using 6 wells plates. Bovine fetal serum (BFS) at 10%, 400 µL of GMCSF and RPMI were added to complete 3mL per well. Figure 5 shows the increase (%) in the molecular markers (CD80, CD86 and CD40) indicative of DCs activation as a result of the exposition of these cells in vitro to both the active and the heat-inactivated StII variants, results comparable to those obtained with the positive control.

### Example 6. Assessment of cytotoxicity and CTL reponse of the vaccinal vehicle using St mutants and OVA, as antigen protein.

The StI dimeric variants of reversibly (StIW111C) or irreversibly (StIW111Cᵢᵣᵣₑᵥ) low pore-forming activity and the thermal inactivated Stll variant were co encapsulated with OVA into liposomes of DPPC:Cho (1:1), in a ratio 10 µmol total lipid: 1.1 nmol OVA: 0.3 nmol of StIW111C, StIW111Cᵢᵣᵣₑᵥ or heat-inactivated Stll, in PBS pH 7.4 and the ability of these vaccine preparations to induce an OVA-specific cytotoxic activity *in vivo* was assessed. In these assays, essentially the same vaccinal compositios were used as those described in Example 1, in the case of composition B, the different variants of St were employed.

Vaccine composition A: DRVs liposomes of DPPC:Cho (60 µmol of total lipids) encapsulating 6.6 nmol of OVA.

Vaccine composition B: DRVs liposomes of DPPC:Cho (60 µmol of total lipids) co-encapsulating 6.6 nmol of OVA and 1.875 nmol of St (StIW111C, StIW111Cᵢᵣᵣₑᵥ, native St II or heat-inactivated Stll)

In a first assay, twelve female mice C57BL/6 were selected with a body weight between 18-20 g, and separated into 4 experimental groups of three animals each.

Group1 (negative control) was inoculated by subcutaneous route, on days 0 and 12, with 0.2 mL of PBS.

Group 2 (positive control) was inoculated by subcutaneous route, on days 0 and 12, with 0.2 mL of the vaccinal composition B (equivalent to 1.1 nmol or 50 µg of OVA and 0.3 nmol or 6.25 µg of native StII).

Group 3 was inoculated by subcutaneous route, on days 0 and 12, with 0.2 mL of the vaccinal composition B (equivalent to 1.1 nmol or 50 µg of OVA and 0.3 nmol or 6.25 µg of StIW111C).

Group 4 was inoculated by subcutaneous route, on days 0 and 12, with 0.2 mL of the vaccinal composition B (equivalent to 1.1 nmol or 50 µg of OVA and 0.3 nmol or 6.25 µg of StIW111Cᵢᵣᵣₑᵥ).

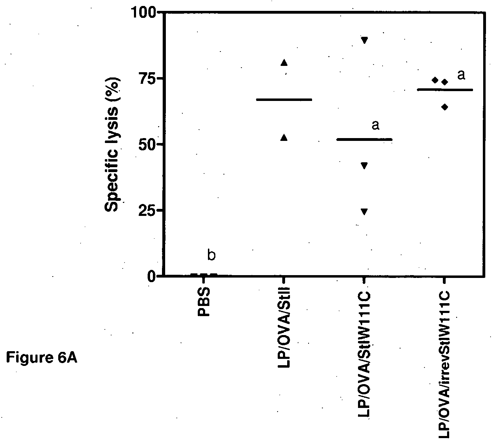

Figura 6A shows that animals immunized with liposomes containing St variants of low pore-forming activity (LP/OVA+StIW111C or LP/OVA+StIW111Cirrev) exhibit a cytotoxic response statistically similar to that obtained with StII when co-encapsulated with OVA into liposomes (LP/OVA+Stll).

In other assay, nine female mice C57BL/6 with a body weight between 18-20 g were selected and separated into 3 groups of 3 animals each.

Group 1 (negative control) was inoculated by subcutaneous route, on days 0 and 12, with 0.2 mL of saline phosphate buffer (PBS).

Group 2 was inoculated by subcutaneous route, on days 0 and 12, with 0.2 mL of vaccinal composition A (equivalent to 1.1 nmol or 50 µg of OVA).

Group 3 was inoculated by subcutaneous route, on days 0 and 12, with 0.2 mL of vaccinal composition B (equivalent to 1.1 nmol or 50 µg of OVA and 0.3 nmol or 6.25 µg of inactive Stll).

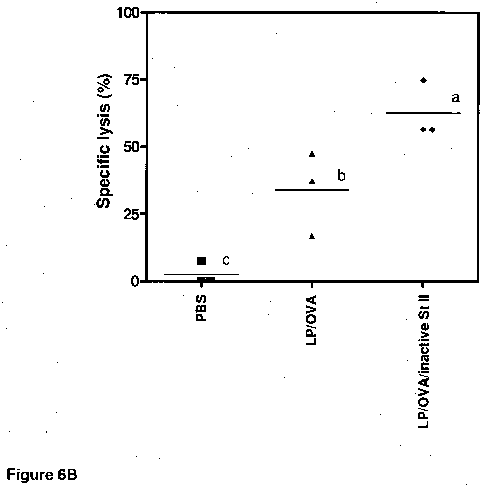

Figure 6B evidences that immunization with liposomes co-encapsulating OVA the completely heat-inactivated Stll variant elicited an OVA-specific cytotoxic activity significantly higher than that induced by liposomes containing only the antigen and similar to that observed with the liposomal formulation containing native Stl/Stll.

The experimental results demonstrated that the liposomal vaccine object of this invention co-encapsulating an antigen with any sticholysin variant, even with those that do not display pore-forming activity, induced a potent, robust and functional antigen-specific CTL response, even larger than that elicited by the classical positive control (PIC) used in an *in vivo* CTL assay. The formulation liposome-St on a preventive scenario significantly increased the time-course of tumor implantation and significantly increase survival in the groups evaluated in relation to those that only received PBS. In summary, vaccination with liposomes-St exhibited better results than those observed with liposomal vesicles only containing the antigen.

### BRIEF DESCRIPTION OF THE DRAWINGS

**Figure 1****:** represents a graphics showing lysis percentage of the target cells loaded with OVA-immunodominat peptide (SIINFEKL) and labeled with CFSE in experimental animals subjected to different vaccine treatments in an *in vivo* cytotocity assay. Each point corresponds to data from a single animal and the line to the mean value of at least two independent experiments. Different letters indicate significant statistical differences among immunized groups according to Dunnett T3 test (p< 0.05).
**Figure 2A****:** represents the percentage of animals free of tumor in three groups of experimental animals subjected to different vaccine treatments and challenged with OVA- expressing tumor cells.
**Figure 2B****:** represents a graphic that allows visualizing the survival parameter in the three mentioned groups after inoculation of the OVA-expressing tumor cells. Different letters indicate significant statistical differences according to the Log-Rank test (p< 0.05).
**Figure 3****:** shows variation in turbidity of an erythrocyte suspension due to the action of dimeric variants of Stl (StIW111C or StIW111Cᵢᵣᵣₑᵥ) under reducing- (in the presence of 2-ME) and no reducing conditions.
**Figure 4****:** represents loss of turbidity of an erythrocyte suspension due to the activity of native StII (active protein) or inactivated by thermal treatment.
**Figure 5****:** shows changes in the expression of molecular markers of dendritic cells (DCs) due to their exposition *in vitro* to StII both in its active and thermal-inactivated variant in the presence or not of an endotoxin neutralizing agent (pmxB).
   Graphic A: Percentage of CD80
   Graphic B: Percentage of CD86
   Graphic C: Percentage of CD40
**Figure 6****:** represents two graphics showing lysis percentage of target cells loaded with OVA-immunodominant peptide (SIINFEKL) and labeled with CFSE in experimental animals subjected to different vaccine treatments in an *in vivo* cytotoxicity assay. Each point corresponds to data from a single animal and the line the mean value. Different letters indicate significant statistical differences among the immunized groups according to the Tukey test (p< 0.05).
   Graphic A: Response obtained upon immunization with liposomes co-encapsulating OVA, native Stll or dimeric variants of Stl reversibly- (StIW111C) or irreversibly (StIW111Cirrev) inactive.
   Graphic B: Response obtained upon immunization with liposomes co-encapsulating OVA and StII inactivated by thermal treatment.

## Claims

1. A vaccine vehicle comprising a co-encapsulation into liposomal vesicles of proteins obtained from the anemone *Stichodactyla helianthus* together with an antigen, wherein the proteins are Sticholysins, and wherein the vaccine vehicle induces a cellular immune response.

2. The vaccine vehicle according to claim 1, wherein the liposomal vesicle contains dipalmitoylphosphatidylcholine (DPPC) and Cho in an equimolar ratio.

3. The vaccine vehicle according to claim 1, wherein the proteins from the anemone *Stichodactyla helianthus* are selected from the group comprising Sticholysin II (StII), Sticholysin I (StI), Sticholysin I mutant W111C (StIW111C), StIW111C irreversibly inactive dimer (StIW111Cᵢᵣᵣₑᵥ) or heat-inactivated StII.

4. The vaccine vehicle according to any one of claims 1-3, wherein the antigen is a protein or a polypeptide associated with a pathology selected from the group comprising cancer and infectious diseases caused by intracellular pathogens.

5. The vaccine vehicle according to claim 4, wherein the antigen is a protein or a polypeptide associated with cancer.

6. The vaccine vehicle according to claim 4, wherein the antigen is a protein or a polypeptide associated with AIDS.

7. The vaccine vehicle according to claim 4, wherein the antigen is a protein or a polypeptide associated with tuberculosis.

8. A vaccine composition comprising the vaccine vehicle according to any one of claims 1-7, said composition lacking other immunological adjuvants.

9. The vaccine vehicle according to any one of claims 1-7, for use in inducing a cellular immune response.

10. The vaccine composition according to claim 8, for use in enhancing the immune response of a patient suffering from a disease selected from the group comprising cancer and infectious diseases caused by intracellular pathogens.

## Patentansprüche

1. Impfstoffvehikel, umfassend eine Co-Einkapselung in liposomale Vesikel von Proteinen, erhalten von der Anemone *Stichodactyla helianthus,* zusammen mit einem Antigen, wobei die Proteine Sticholysine sind und wobei das Impfstoffvehikel eine zelluläre Immunreaktion induziert.

2. Impfstoffvehikel nach Anspruch 1, wobei das liposomale Vesikel Dipalmitoylphosphatidylcholin (DPPC) und Cho in einem äquimolaren Verhältnis enthält.

3. Impfstoffvehikel nach Anspruch 1, wobei die Proteine von der Anemone *Stichodactyla helianthus* ausgewählt sind aus der Gruppe umfassend Sticholysin II (StII), Sticholysin I (StI), Sticholysin I Mutante W111C (StIW111C), StIW111C irreversibel inaktives Dimer (StIW111Cᵢᵣᵣₑᵥ) oder hitzeinaktiviertes StII.

4. Impfstoffvehikel nach einem der Ansprüche 1-3, wobei das Antigen ein Protein oder ein Polypeptid ist, assoziiert mit einer Pathologie, ausgewählt aus der Gruppe umfassend Krebs und Infektionskrankheiten, verursacht durch intrazelluläre Krankheitserreger.

5. Impfstoffvehikel nach Anspruch 4, wobei das Antigen ein Protein oder ein Polypeptid, assoziiert mit Krebs, ist.

6. Impfstoffvehikel nach Anspruch 4, wobei das Antigen ein Protein oder ein Polypeptid, assoziiert mit AIDS, ist.

7. Impfstoffvehikel nach Anspruch 4, wobei das Antigen ein Protein oder ein Polypeptid, assoziiert mit Tuberkulose, ist.

8. Impfstoffzusammensetzung, umfassend das Impfstoffvehikel nach einem der Ansprüche 1-7, wobei der Zusammensetzung andere immunologische Hilfsstoffe fehlen.

9. Impfstoffvehikel nach einem der Ansprüche 1-7 zur Verwendung bei dem Induzieren einer zellulären Immunreaktion.

10. Impfstoffzusammensetzung nach Anspruch 8, zur Verwendung bei der Verbesserung der Immunreaktion eines Patienten, der an einer Krankheit leidet, ausgewählt aus der Gruppe umfassend Krebs und Infektionskrankheiten, verursacht durch intrazelluläre Krankheitserreger.

## Revendications

1. Véhicule vaccinal comprenant un co-encapsulation dans des vésicules liposomales de protéines obtenues à partir de l'anémone *Stichodactyla helianthus* conjointement avec un antigène, dans lequel les protéines sont des Sticholysines, et dans lequel le véhicule vaccinal induit une réponse immunitaire cellulaire.

2. Véhicule vaccinal selon la revendication 1, dans lequel la vésicule liposomale contient de la dipalmitoylphosphatidylcholine (DPPC) et des Cho dans un rapport équimolaire.

3. Véhicule vaccinal selon la revendication 1, dans lequel les protéines de *Stichodactyla helianthus* anémone sont choisies dans le groupe comprenant Sticholysine II (StII), Sticholysine I (StI), mutant W111C de Sticholysine I (StIW111C), dimère irréversiblement inactif de StIW111C (StIW111Cᵢᵣᵣₑᵥ) ou StII inactivée par la chaleur.

4. Véhicule vaccinal selon l'une quelconque des revendications 1 à 3, dans lequel l'antigène est une protéine ou un polypeptide associé(e) à une pathologie choisie dans le groupe comprenant le cancer et les maladies infectieuses provoquées par des pathogènes intracellulaires.

5. Véhicule vaccinal selon la revendication 4, dans lequel l'antigène est une protéine ou un polypeptide associé(e) au cancer.

6. Véhicule vaccinal selon la revendication 4, dans lequel l'antigène est une protéine ou un polypeptide associé(e) au SIDA.

7. Véhicule vaccinal selon la revendication 4, dans lequel l'antigène est une protéine ou un polypeptide associé(e) à la tuberculose.

8. Composition vaccinale comprenant le véhicule vaccinal selon l'une quelconque des revendications 1 à 7, ladite composition étant dépourvue d'autres adjuvants immunologiques.

9. Véhicule vaccinal selon l'une quelconque des revendications 1 à 7, destiné à être utilisé afin d'induire une réponse immunitaire cellulaire.

10. Composition vaccinale selon la revendication 8, destinée à être utilisée dans le renforcement de la réponse immunitaire d'un patient souffrant d'une maladie choisie dans le groupe comprenant le cancer et les maladies infectieuses provoquées par des pathogènes intracellulaires.
